# EUROPÄISCHE PATENTANMELDUNG

(11) **EP 0 546 291 A1**
(43) Veröffentlichungstag der Anmeldung: **16.06.1993**
(21) Anmeldenummer: 92118137.6
(22) Anmeldetag: 23.10.1992
(51) Int. Cl.: G01N 27/49, G01N 33/00

(54) **Elektrochemische Messzelle zur Bestimmung von Ammoniak oder Hydrazin in einer Messprobe**

(30) Priorität: 11.12.1991 US 804722; 22.08.1992 DE 4227946
(71) Anmelder: Drägerwerk Aktiengesellschaft, D-23542 Lübeck (DE)
(72) Erfinder: Kiesele, Herbert, Dr., W-2400 Lübeck (DE); Kühn, Uwe, W-2067 Wesenberg (DE); Haupt, Stephan, Dr., W-2400 Lübeck (DE)

(57) **Zusammenfassung**

Eine elektrochemische Meßzelle für Ammoniak oder Hydrazin mit mindestens einer Meßelektrode und einer Gegenelektrode in einem Elektrolyt soll so verbessert werden, daß eine Signalerhöhung mit einer kürzeren Ansprechzeit und einer besseren Signalstabilität gewonnen wird. Dazu ist vorgesehen, daß der Elektrolyt aus einer wäßrigen Lösung eines hygroskopischen Salzes eines Alkali- oder Erdalkalimetalles, z.B. von Kalziumnitrat oder Lithiumnitrat, oder einer Mischung aus beiden Salzen besteht.

## Beschreibung

Die Erfindung betrifft eine elektrochemische Meßzelle zur amperometrischen Bestimmung von Ammoniak oder Hydrazin in einer gasförmigen oder flüssigen Meßprobe, mit mindestens einer Meßelektrode und einer Gegenelektrode, die in einer mit einem löslichen Elektrolyten gefüllten Elektrolytkammer aufgenommen sind, welche zur Meßprobe hin durch eine permeable Membran abgeschlossen ist.

Eine derartige elektrochemische Meßzelle ist aus der EP-A 395 927 bekanntgeworden.

Bei der bekannten Meßzelle diffundieren die NH₃-Moleküle aus der Gasphase durch die poröse Membran und einen dünnen Elektrolytfilm an die Elektrode und werden dort anodisch oxidiert. Wesentlich für eine hohe Empfindlichkeit des Sensors ist die Permeabilität des Meßgases im Elektrolytfilm. Die minimale Arbeitstemperatur des Sensors wird durch den Gefrierpunkt des Elektrolyten begrenzt, während die Lebensdauer des Sensors maßgeblich durch den Wasserdampfdruck des Elektrolyten bestimmt wird.

Der vorliegenden Erfindung liegt somit die Aufgabe zugrunde, eine Meßzelle der genannten Art so zu verbessern, daß die Empfindlichkeit erhöht, die minimale Arbeitstemperatur erniedrigt und die Lebensdauer vergrößert wird.

Die Lösung der Aufgabe erfolgt dadurch, daß als Elektrolyt eine wäßrige Lösung eines hygroskopischen Salzes eines Alkali- oder Erdalkalimetalles oder einer Mischung aus beiden Salzen vorgesehen ist, die einen neutralen bis leicht sauren pH-Wert des Elektrolyten ergeben.

Die hygroskopischen Salze bewirken eine Gefrierpunktserniedrigung, so daß die Sensoren bis ca. -50 _{°} C in Kühlhäusern oder im Freiland eingesetzt werden können. Der Wasserdampfdruck der Elektrolytlösung wird dabei erniedrigt, so daß sich der Flüssigkeitsverlust verlangsamt und auf ein großes Elektrolytreservoir verzichtet werden kann.

Unter hygroskopischen Salzen sollen solche verstanden werden, die in einer abgeschlossenen Umgebung die relative Luftfeuchte unterhalb 65% bei Normaltemperatur absenken. Wählt man als Beispiel eines hygroskopischen Salzes Lithiumnitrat, Magnesiumnitrat oder Calciumnitrat, oder ein Gemisch dieser Salze, so bilden Ammoniak und Hydrazin mit den Kationen Komplexe bzw. komplexartige Verbindungen. Hierdurch vergrößert sich die Löslichkeit bzw. die Permeabilität und somit die Empfindlichkeit.

Neben den genannten Nitraten können als Salze ebenso mit dem gleichen Ergebnis Chloride oder Bromide eingesetzt werden.

Die Lösung der Salze im Elektrolyten wird vorzugsweise in einer Konzentrationshöhe von 3 Molar und größer vorgenommen. Dies führt einerseits zu dem gewünschten pH-Wert, und andererseits wird der Gefrierpunkt genügend erniedrigt.

Zur weiteren Verbesserung der elektrochemischen Meßzelle ist es günstig, die Meßelektrode mit einer Beschichtung zu versehen, welche Kobaltoxid enthält, wobei die Beschichtung auf der Meßelektrode so aufgebracht ist, daß sie in direktem Kontakt mit dem Elektrolyten steht. Durch die Kobaltoxid-Beschichtung wird ein verbesserter Schutz der Meßelektrodenoberfläche vor Störreaktion von Reaktionssprodukten erzielt.

Wird eine sogenannte Drei-Elektroden-Meßzelle benutzt, ist es zweckmäßig, eine Referenzelektrode in dem Elektrolyten vorzusehen, die ebenfalls eine Beschichtung aufweist, welche Kobaltoxid enthält.

Meßelektrode und Referenzelektrode können vorzugsweise aus einem Edelmetall, z.B. Gold, bestehen, wobei die Beschichtung elektrolytisch auf dem Trägermaterial der Elektrode abgeschieden ist.

Es kann ebenso günstig sein, die Meßelektrode und die Referenzelektrode aus einem Träger herzustellen, der aus einer Kobaltlegierung besteht und wobei die Beschichtung aus einer Oxidschicht gebildet ist, welche durch Oxidieren der Legierung gewonnen wurde.

Bei einem Elektrodenmaterial mit einem Kobaltoxidüberzug ist es zweckmäßig, dem Elektrolyten ein lösliches Kobaltsalz zuzusetzen. Das Kobaltsalz wirkt als Katalysator, indem es den Kobaltoxidüberzug regeneriert, sollte er während des Betriebs der Meßzelle beschädigt werden. Darüber hinaus unterstützt das Kobaltsalz in dem Elektrolyten die katalytische Reaktion der Ammoniak- oder Hydrazin-Oxidation an der Meßelektrode. Dadurch werden die Empfindlichkeit und die Ansprechgeschwindigkeit der chemischen Meßzelle erhöht.

Ein günstiges Kobaltsalz zu diesem Zwecke ist das Kobaltnitrat. Durch die Verwendung des Kobaltsalzes, insbesondere des Kobaltnitrates, wird die Erzeugung einer neuen Kobaltoxidschicht begünstigt, sofern sie durch eine chemische Störreaktion angegriffen wurde. Eine besonders geeignete Mischung für den Elektrolyten ist eine 3,5 molare Lösung Kalziumnitrat oder Lithiumnitrat und ein 0,1 millimolarer Kobaltnitratzusatz als Katalysator. Hierbei kann der Zusatz an Kobaltnitrat bis auf 1 Millimol erhöht werden, ohne daß die Nachweisfunktion für die Meßzelle beeinträchtigt wird.

Für die elektrochemische Meßzelle ist deren fehlende Querempfindlichkeit gegen Kohlenmonoxid und Wasserstoff hervorzuheben. Um für die Bestimmung von Ammoniak oder Hydrazin ein

Referenzpotential zu erzeugen, wird in die Meßzelle eine Referenzelektrode eingebracht, deren Potential als Bezugspunkt für die Messung steht. Es ist zweckmäßig, eine solche Referenzelektrode ebenfalls mit einem kobaltoxidhaltigen Überzug zu versehen. Eine derartige Meßzelle hat den Vorteil, daß sie mit kurzgeschlossenen Elektroden lagerfähig ist, wodurch sie wegen der kleinen Einlaufzeit sofort betriebsbereit wird. Außerdem wird die Abhängigkeit des Grundstromes von der Temperatur minimiert, da das Potential der Meßelektrode und der Referenzelektrode in gleicher Weise von der Temperatur beeinflußt werden.

Ein Ausführungsbeispiel der Erfindung wird anhand einer schematischen Zeichnung dargestellt und im folgenden näher erläutert.

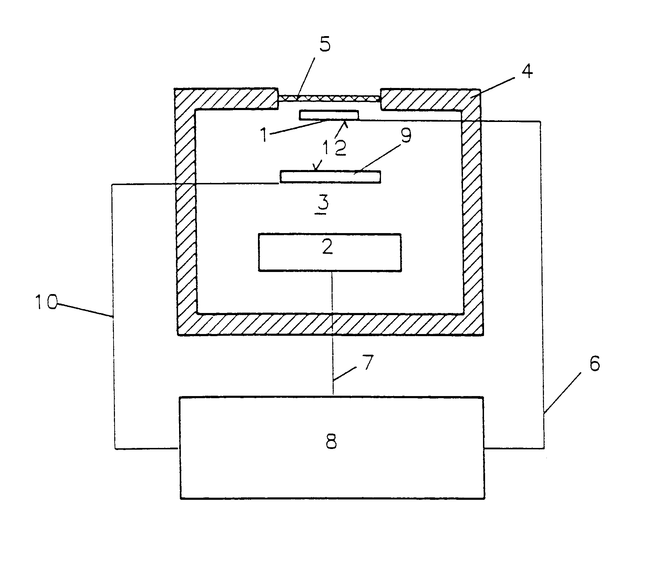

Die einzige Figur zeigt eine elektrochemische Meßzelle mit einem Elektrolyten (3) aus einer wäßrigen Lösung von Kalziumnitrat und Lithiumnitrat. Der Elektrolyt ist in einem Gehäuse (4) enthalten und in ihm sind eine Meßelektrode (1), eine Gegenelektrode (2) und eine Referenzelektrode (9) eingebracht. Die Elektroden (1, 9) besitzen einen kobaltoxidhaltigen Überzug (12). Zur die Meßprobe enthaltenden Umgebung hin ist der Elektrolyt (3) durch eine für Ammoniak und Hydrazin permeable Membran (5) abgeschlossen, welche an dem Gehäuse (4) dichtend befestigt ist. Die Meßelektrode (1), die Gegenelektrode (2) und die Referenzelektrode (9) besitzen Meßanschlüsse (6, 7, 10), welche durch das Gehäuse (4) hindurchgeführt und an eine Auswerteeinheit (8) zur weiteren Verarbeitung der Meßsignale angeschlossen sind. Meßelektrode (1) und Gegenelektrode (2) werden durch die Auswerteeinheit (8) mit einer Arbeitsspannung von etwa 600 mV versorgt.

## Patentansprüche

1. Elektrochemische Meßzelle zur amperometrischen Bestimmung von Ammoniak oder Hydrazin in einer gasförmigen oder flüssigen Meßprobe, mit mindestens einer Meßelektrode und einer Gegenelektrode, die in einer mit einem löslichen Elektrolyten gefüllten Elektrolytkammer aufgenommen sind, welche zur Meßprobe hin durch eine permeable Membran abgeschlossen ist, dadurch gekennzeichnet, daß als Elektrolyt (3) eine wäßrige Lösung eines hygroskopischen Salzes eines Alkali-oder Erdalkalimetalls oder einer Mischung aus beiden Salzen vorgesehen ist, die einen neutralen bis leicht sauren pH-Wert im Elektrolyten (3) ergeben.

2. Elektrochemische Meßzelle nach Anspruch 1, dadurch gekennzeichnet, daß der kationische Bestandteil des Salzes aus Lithium oder Magnesium oder Calcium, und der anionische Bestandteil aus einem Nitrat oder Chlorid oder Bromid besteht.

3. Elektromagnetische Meßzelle nach einem der Ansprüche 1 bis 3, dadurch gekennzeichnet, daß die Lösung der hygroskopischen Salze in einer Konzentration größer 3 Molar vorliegen.

4. Elektrochemische Meßzelle nach Anspruch 1, 2 oder 3, dadurch gekennzeichnet, daß die Meßelektrode (1) einen Überzug (12) enthält, der aus Kobaltoxid besteht und daß der Überzug (12) auf der Meßelektrode (1) so gebildet ist, daß er in direktem Kontakt mit dem Elektrolyten (3) steht.

5. Elektrochemische Meßzelle nach Anspruch 4, dadurch gekennzeichnet, daß sie eine Referenzelektrode (9) in dem Elektrolyten (3) enthält, welche einen Überzug (12) aus Kobaltoxid besitzt.

6. Elektrochemische Meßzelle nach Anspruch 4 oder 5, dadurch gekennzeichnet, daß zumindest eine der Meßelektrode (1) oder der Referenzelektrode (9) jeweils aus einem Träger aus einem Edelmetall besteht und daß der Überzug (12) elektrolytisch auf dem Träger abgeschieden ist.

7. Elektrochemische Meßzelle nach Anspruch 4 oder 5, dadurch gekennzeichnet, daß zumindest die Meßelektrode (1) oder die Referenzelektrode (9) aus einem Träger bestehen, der eine Kobaltlegierung enthält, die zur Bildung eines kobaltoxidhaltigen Überzugs (12) oxidiert worden ist.

8. Elektrochemische Meßzelle nach einem der Ansprüche 1 bis 7, dadurch gekennzeichnet, daß in dem Elektrolyten (3) ein Kobaltsalz gelöst ist.

9. Elektrochemische Meßzelle nach Anspruch 8, dadurch gekennzeichnet, daß das Kobaltsalz ein Kobaltnitrat ist.
